# EUROPEAN PATENT APPLICATION

(11) **EP 4 410 354 A1**
(43) Date of publication of application: **07.08.2024**
(21) Application number: 22895219.8
(22) Date of filing: 14.09.2022
(51) Int. Cl.: A61M 39/10

(54) **MEDICAL CONNECTOR**

(30) Priority: 17.11.2021 JP 2021187406
(71) Applicant: TERUMO Kabushiki Kaisha, Tokyo 151-0072 (JP)
(72) Inventor: KURIYAMA Tasuku, Nakakoma-gun, Yamanashi 409-3853 (JP)
(74) Representative: TBK
(86) International application number: PCT/JP2022/034474
(87) International publication number: WO 2023/089933

(57) **Abstract**

A medical connector including: a first connection member having a first connection port and a second connection port that fluidically communicates with the first connection port; a second connection member; and a ratchet mechanism portion that couples the first connection member and the second connection member, in which a first connection operation by rotation is applied to the second connection member to allow the first connection member to co-rotate via the ratchet mechanism portion to allow the first connection port to be fluidically connected and allow the first connection member to be mechanically connected to the first medical device, the second connection member is mechanically connected to a second medical device by a second connection operation to allow the second connection port to be fluidically connected to the second medical device without intervention of the second connection member, and an operation in a direction opposite to the first connection operation is applied to the second connection member to allow the second connection member to idle with respect to the first connection member via the ratchet mechanism.

## Description

### Technical Field

The present disclosure relates to a medical connector.

### Background Art

There has been known a medical connector including: a first connection member having a first connection port and a second connection port that fluidically communicates with the first connection port; and a second connection member, in which a first connection operation by rotation is applied to the second connection member to allow the first connection port to be fluidically connected and allow the first connection member to be mechanically connected to a first medical device, and the second connection member is mechanically connected to a second medical device by a second connection operation to allow the second connection port to be fluidically connected to the second medical device without intervention of the second connection member (see, for example, Patent Literature 1).

### Citation List

### Patent Literature

Patent Literature 1: JP 2021-106791 A

### Summary of Invention

### Technical Problem

It is desirable that the medical connector as described above is less easily detached from the first medical device when an operation in a direction opposite to the first connection operation is unintentionally applied to the second connection member while being connected to the first medical device.

Therefore, an object of the present disclosure is to provide a medical connector that is less easily detached unintentionally from the first medical device.

### Solution to Problem

As one aspect of the present disclosure, a medical connector is a medical connector including: a first connection member having a first connection port and a second connection port that fluidically communicates with the first connection port; a second connection member; and a ratchet mechanism portion that couples the first connection member and the second connection member, in which a first connection operation by rotation is applied to the second connection member to allow the first connection member to co-rotate via the ratchet mechanism portion to allow the first connection port to be fluidically connected and allow the first connection member to be mechanically connected to the first medical device, the second connection member is mechanically connected to a second medical device by a second connection operation to allow the second connection port to be fluidically connected to the second medical device without intervention of the second connection member, and an operation in a direction opposite to the first connection operation is applied to the second connection member to allow the second connection member to idle with respect to the first connection member via the ratchet mechanism.

As one embodiment of the present disclosure, the medical connector is a medical connector in which the first connection member includes a female connector having the first connection port.

As one embodiment of the present disclosure, the medical connector is a medical connector in which the first connection member includes a spike having the second connection port.

As an embodiment of the present disclosure, the medical connector is a medical connector including a valve body having a head portion and a body portion, the head portion being movable by elastic deformation of the body portion in a longitudinal direction of the spike between a closing position where the second connection port is closed and an opening position where the second connection port is opened.

As an embodiment of the present disclosure, the medical connector is a medical connector including an engagement claw portion that is pushed and elastically deformed radially inward of the spike by moving integrally with the head portion of the valve body on a root side of the spike and engages with the second medical device.

As one embodiment of the present disclosure, the medical connector is a medical connector in which the second connection member has a locking claw portion capable of being mechanically connected to the second medical device by elastic deformation.

### Advantageous Effects of Invention

According to the present disclosure, it is possible to provide a medical connector that is less easily detached unintentionally from the first medical device.

### Brief Description of Drawings

Fig. 1 is a longitudinal cross-sectional view illustrating a medical connector according to one embodiment.
Fig. 2 is a longitudinal cross-sectional view when viewed from a direction 90° different from that in Fig. 1.
Fig. 3 is a perspective view of the medical connector illustrated in Fig. 1.
Fig. 4 is a perspective view illustrating a first connection member illustrated in Fig. 1.
Fig. 5 is a perspective view illustrating a holder illustrated in Fig. 1.
Fig. 6 is a cross-sectional view illustrating a state in which a first medical device and a second medical device are connected to the medical connector illustrated in Fig. 1.
Fig. 7 is a longitudinal cross-sectional view when viewed from a direction 90° different from that in Fig. 6.

### Description of Embodiment

An embodiment of the present disclosure will be described in detail below with reference to the drawings.

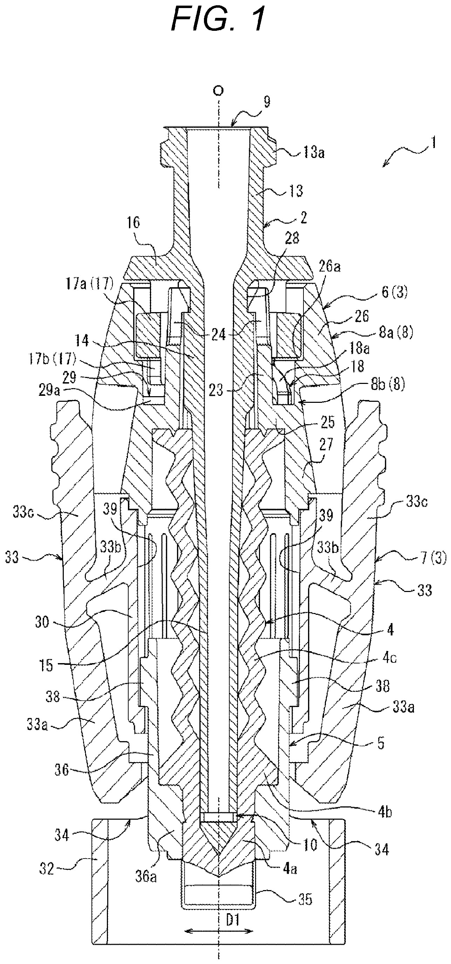

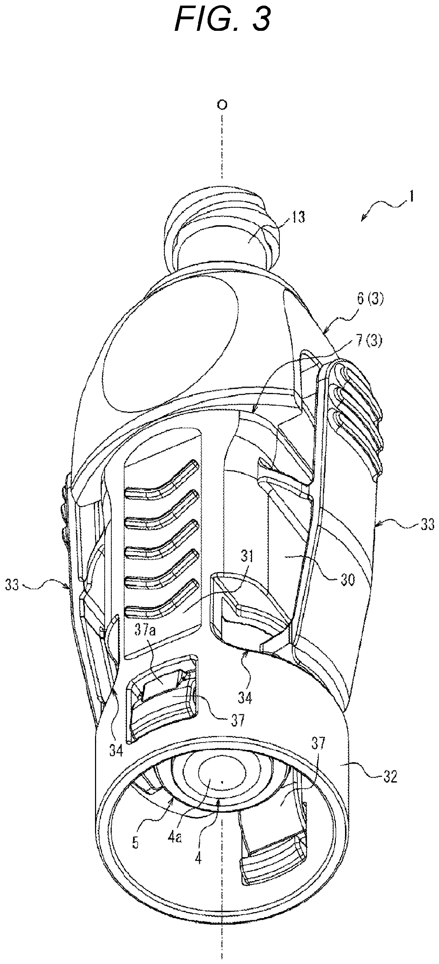

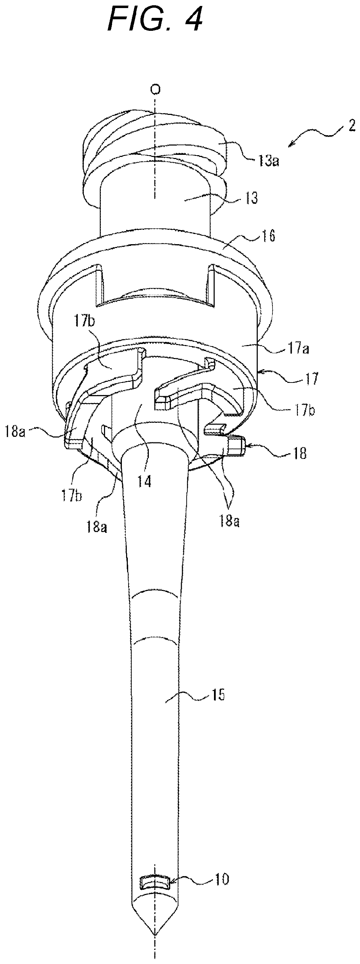

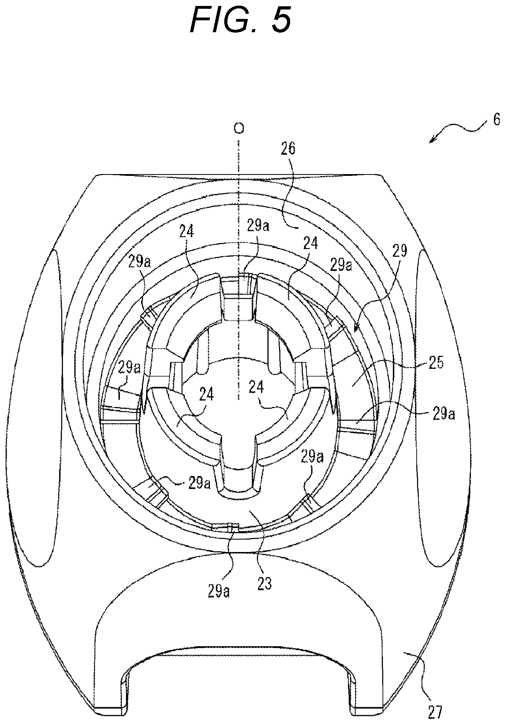

As illustrated in Figs. 1 to 5, a medical connector 1 according to one embodiment of the present disclosure includes a first connection member 2, a second connection member 3, a valve body 4, and an engagement member 5. The second connection member 3 includes a holder 6 and a second connection member body 7. In addition, the medical connector 1 includes a ratchet mechanism portion 8 that couples the first connection member 2 and the second connection member 3. The ratchet mechanism portion 8 includes a rotation coupling portion 8a and a rotation regulating portion 8b. The medical connector 1 can be used, for example, to form a liquid delivery line for infusion solution or the like to a living body such as a patient. The medical connector 1 is configured as an I-shaped connector.

Each of the first connection member 2, the holder 6, the second connection member body 7, and the engagement member 5 is made of, for example, synthetic resin. The valve body 4 is made of, for example, elastomer or rubber. Each of the first connection member 2, the holder 6, the second connection member body 7, and the engagement member 5 is formed by, for example, injection molding. Note that the configuration of the second connection member 3 is not limited to the configuration including two components of the holder 6 and the second connection member body 7, but may be a configuration including one component or a configuration including three or more components.

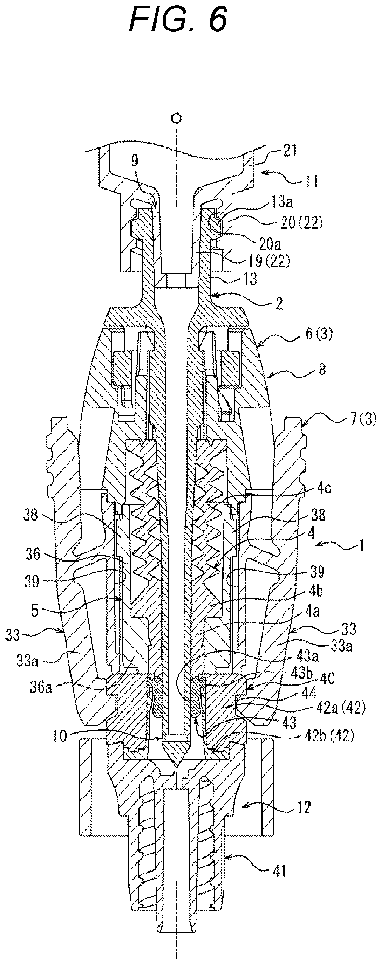

In addition, the first connection member 2 has a first connection port 9 and a second connection port 10 that fluidically communicates with the first connection port 9. As illustrated in Figs. 6 to 7, the first connection port 9 can be fluidically and mechanically connected to a first medical device 11. By mechanically connecting the second connection member 3 to a second medical device 12, the second connection port 10 can be fluidically connected to the second medical device 12 without intervention of the second connection member 3.

The first medical device 11 is configured as a syringe. The second medical device 12 is configured as an I-shaped connector.

In the present embodiment, for convenience of description, regarding the medical connector 1, a direction along a central axis O is also referred to as an up-and-down direction, a direction from the first connection port 9 toward the second connection port 10 is also referred to as a downward direction, the opposite direction of the downward direction is also referred to as an upward direction, a direction along a straight line perpendicular to the central axis O is also referred to as a radial direction, a direction around the central axis O is also referred to as a circumferential direction, and a cross section taken along a plane including the central axis O is also referred to as a longitudinal cross section. In addition, the first medical device 11 and the second medical device 12 will be described with reference to a direction at the time of connection to the medical connector 1. Note that the medical connector 1 may be disposed and used in an arbitrary direction with respect to the vertical direction.

As illustrated in Figs. 1 to 4, the first connection member 2 includes a female connector 13, a hollow shaft portion 14, a spike 15, a flange portion 16, an outer wall portion 17, and an elastic deformation portion 18.

The female connector 13 has a cylindrical shape centered on the central axis O, and has the first connection port 9 at the upper end portion. In addition, the inner circumferential surface of the female connector 13 has, for example, a fitting surface by a standardized Luer taper. The outer circumferential surface of the female connector 13 has a male screw portion 13a that can be screwed into the first medical device 11.

The first medical device 11 includes an inner cylindrical body 19 having an outer circumferential surface that can be fitted to the inner circumferential surface of the female connector 13, and an outer cylindrical body 20 having a female screw portion 20a that can be screwed into the male screw portion 13a of the female connector 13. The inner circumferential surface of the inner cylindrical body 19 forms a part of the internal passage of the first medical device 11, more specifically, a passage at the distal end of a syringe body 21 to which a pusher is fitted. The inner cylindrical body 19 and the outer cylindrical body 20 constitute a male connector 22 that can be fluidically and mechanically connected to the female connector 13.

The male screw portion 13a is configured as a right screw, and can be screwed into the female screw portion 20a by relatively rotating clockwise in a bottom view with respect to the female screw portion 20a, and can be screwed out from the female screw portion 20a by relatively rotating counterclockwise in a bottom view with respect to the female screw portion 20a.

The hollow shaft portion 14 extends in the downward direction from the lower end of the female connector 13 and has a circular cylindrical shape centered on the central axis O. Note that, a configuration may be adopted in which, the hollow shaft portion 14 has a cylindrical shape other than a circular cylindrical shape, such as a square cylindrical shape. The inner circumferential surface of the hollow shaft portion 14 forms a passage.

The spike 15 extends in the downward direction from the lower end of the hollow shaft portion 14, has a tapered bottomed cylindrical shape centered on the central axis O, and has the second connection port 10. The passage formed by the inner surface of the spike 15 communicates with the second connection port 10 constituted by two through-holes penetrating the distal end portion of the spike 15 in the radial direction. Note that the number of through-holes constituting the second connection port 10 is not limited to two.

The flange portion 16 extends radially outward from the upper end portion of the hollow shaft portion 14 and has a circular ring shape centered on the central axis O. Note that, a configuration may be adopted in which, the flange portion 16 has a ring shape other than a circular ring shape, such as a rectangular ring shape.

The outer wall portion 17 extends in the downward direction from the flange portion 16 and forms a gap in the radial direction with the outer circumferential surface of the hollow shaft portion 14. The outer wall portion 17 has an upper wall portion 17a and four lower wall portions 17b. The upper wall portion 17a has a lower end surface extending in the downward direction from the lower surface of the flange portion 16 and continuously extending over the entire circumference. The four lower wall portions 17b are provided side by side in the circumferential direction, and each extends in the downward direction from the lower end surface of the upper wall portion 17a.

The elastic deformation portion 18 is constituted by four elastic projecting pieces 18a. Each of the elastic projecting pieces 18a protrudes counterclockwise in bottom view from a corresponding lower wall portion 17b, and extends obliquely in the downward direction toward the distal end side. The lower surface of the distal end portion of each of the elastic projecting pieces 18a is positioned in the downward direction more than the lower end surface of the corresponding lower wall portion 17b is, and can be displaced in the upward direction by the elastic deformation of the elastic projecting piece 18a.

Note that the number of the elastic projecting pieces 18a constituting the elastic deformation portion 18 is not limited to four, and may be one or two or more other than four. The number of the lower wall portions 17b can be appropriately set according to the number of elastic projecting pieces 18a. Without providing the lower wall portions 17b, a configuration in which each elastic projecting piece 18a protrudes from the upper wall portion 17a may be adopted.

As illustrated in Figs. 1 to 3 and 5, the holder 6 includes an inner circumferential wall 23, four claw pieces 24, a flange wall 25, an upper wall 26, and a lower wall 27.

The inner circumferential wall 23 has a cylindrical shape centered on the central axis O, and the inner circumferential surface of the inner circumferential wall 23 faces the outer circumferential surface of the hollow shaft portion 14. The four claw pieces 24 are provided side by side in the circumferential direction, and each has a shape that protrudes in the upward direction from the upper end of the inner circumferential wall 23 and bends radially inward. The outer circumferential surface of the hollow shaft portion 14 has a circumference groove 28 continuously extending over the entire circumference in a gap portion in the radial direction between the outer wall portion 17 and the hollow shaft portion 14. Each of the claw pieces 24 is locked to the circumference groove 28 to restrict the movement of the second connection member 3 in the downward direction relative to the first connection member 2.

The flange wall 25 extends radially outward from the lower end portion of the inner circumferential wall 23 and has a ring shape centered on the central axis O. The upper wall 26 protrudes in the upward direction from the outer circumferential edge portion of the upper surface of the flange wall 25, and the upper end portion of the upper wall 26 faces the outer circumferential edge portion of the lower surface of the flange portion 16. In addition, the inner circumferential surface of the upper wall 26 has a stepped surface 26a facing the outer circumferential edge portion of the lower end surface of the upper wall portion 17a of the outer wall portion 17. The upper wall 26 restricts the relative movement in the upward direction of the second connection member 3 with respect to the first connection member 2 by the contact between the upper end portion of the upper wall 26 and the outer circumferential edge portion of the lower surface of the flange portion 16 or the contact between the stepped surface 26a of the upper wall 26 and the outer circumferential edge portion of the lower end surface of the outer wall portion 17, or by both of the contacts. Note that, a configuration may be adopted in which, instead of the contact by the upper wall 26, the upper end portions of the four claw pieces 24 contact the lower surface of the flange portion 16 to restrict the relative movement in the upward direction of the second connection member 3 with respect to the first connection member 2.

The four claw pieces 24, the flange wall 25, and the upper wall 26 are fitted to the hollow shaft portion 14, the flange portion 16, and the outer wall portion 17 so that the relative movement in the upward direction and the downward direction is restricted and the rotation in the circumferential direction is allowed. That is, the rotation coupling portion 8a includes the inner circumferential wall 23, the four claw pieces 24, the flange wall 25, the upper wall 26, the hollow shaft portion 14, the flange portion 16, and the outer wall portion 17, and couples the second connection member 3 to the first connection member 2 such that the relative movement in the upward direction and the downward direction is restricted and the rotation in the circumferential direction is allowed.

The upper surface of the flange wall 25 faces the lower surface of the lower wall portion 17b. The upper surface of the flange wall 25 has a ring-shaped uneven surface 29 that has a ring shape centered on the central axis O and has an uneven shape when viewed in the radial direction. The ring-shaped uneven surface 29 is provided so as to abut against the distal end portion of the elastic projecting piece 18a. The ring-shaped uneven surface 29 has eight protrusions 29a provided side by side in the circumferential direction. The number of the protrusions 29a constituting the ring-shaped uneven surface 29 is not limited to 8, and can be appropriately set. When rotating counterclockwise with respect to the elastic deformation portion 18 in a bottom view, each of the protrusions 29a can push up and climb over the distal end portion when abutting against the distal end portion of the adjacent elastic projecting piece 18a. On the other hand, when rotating clockwise with respect to the elastic deformation portion 18 in a bottom view, each of the protrusions 29a cannot push up and climb over the distal end portion when abutting against the distal end portion of the adjacent elastic projecting piece 18a. As described above, the ring-shaped uneven surface 29 can co-rotate clockwise in a bottom view with respect to the elastic deformation portion 18, and can idle counterclockwise due to the elastic deformation of the elastic deformation portion 18. That is, the rotation regulating portion 8b includes the ring-shaped uneven surface 29 and the elastic deformation portion 18, and allows the first connection member 2 to co-rotate clockwise in a bottom view with respect to the second connection member 3 while allowing the first connection member 2 to idle counterclockwise.

Therefore, by allowing the first connection member 2 to co-rotate via the ratchet mechanism portion 8 by applying the first connection operation to the second connection member 3 by the relative rotation clockwise in the bottom view with respect to the first medical device 11, as illustrated in Figs. 6 to 7, the first connection port 9 can be fluidically connected to the internal passage of the male connector 22 of the first medical device 11, and the first connection member 2 can be mechanically connected to the male connector 22 of the first medical device 11. Note that the first medical device 11 is not limited to a syringe, and may be configured as, for example, another medical device having the male connector 22.

The lower wall 27 extends in the downward direction from the outer circumferential edge portion of the flange wall 25. The second connection member body 7 is integrally attached to the lower end portion of the lower wall 27. As illustrated in Figs. 1 to 3, the second connection member body 7 includes an upper cylindrical portion 30, an intermediate coupling portion 31, a lower cylindrical portion 32, and two operation pieces 33. The upper end portion of the upper cylindrical portion 30 is integrally attached to the lower end portion of the lower wall 27 by, for example, welding.

The upper cylindrical portion 30 has a flat cylindrical shape centered on the central axis O and having a width in a first direction D1 smaller than a width in a second direction D2 perpendicular to the first direction D1 in a bottom view. The lower cylindrical portion 32 has a cylindrical shape centered on the central axis O. The upper cylindrical portion 30 and the lower cylindrical portion 32 are integrally continuous with each other by the intermediate coupling portion 31 at two positions facing each other in the second direction D2. Between the upper cylindrical portion 30 and the lower cylindrical portion 32, openings 34 are formed at two positions facing each other in the first direction D1.

The operation pieces 33 are provided integrally continuously in the upper cylindrical portion 30 at two positions facing each other in the first direction D1. Each of the operation pieces 33 includes a locking claw portion 33a extending to the lower side and having a distal end portion (lower end portion) bent radially inward, a coupling portion 33b integrally connecting an upper end portion of the locking claw portion 33a to an outer circumferential surface of the upper cylindrical portion 30, and an operation portion 33c extending to the upper side from the upper end portion of the locking claw portion 33a. By pressing the operation portion 33c radially inward, the entire operation portion 33c and the locking claw portion 33a are inclined with the coupling portion 33b as a fulcrum by elastic deformation, so that the distal end portion of the locking claw portion 33a can be displaced radially outward. The distal end portion of the locking claw portion 33a extends into the corresponding opening 34.

The inner circumferential surface of the lower cylindrical portion 32 has hole portions 35 at two positions facing each other in the second direction D2. Each of the hole portions 35 is configured as a through-hole penetrating the lower cylindrical portion 32. Each of the hole portions 35 is not limited to the through-hole, and may be configured as a recessed (bottomed hole-shaped) hole portion 35.

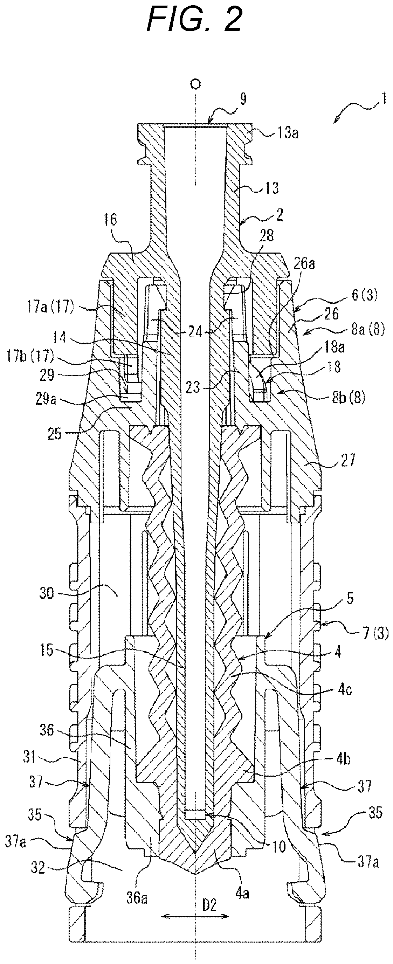

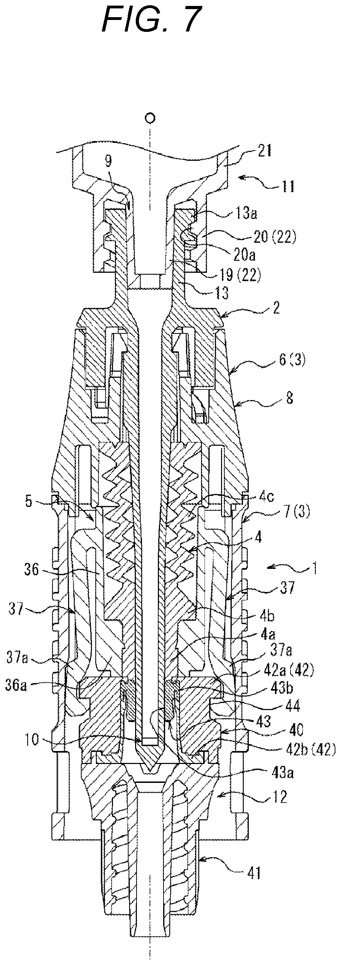

The valve body 4 has a head portion 4a, a shoulder portion 4b, and a body portion 4c. The head portion 4a has a bottomed cylindrical shape, and the head portion 4a closes the second connection port 10 of the spike 15 at the closing position illustrated in Figs. 1 and 2 and opens the second connection port 10 at the opening position illustrated in Figs. 6 and 7. The shoulder portion 4b is continuous with the upper end of the head portion 4a and protrudes radially outward from the head portion 4a. The body portion 4c extends in the upward direction from the upper end of the shoulder portion 4b and has a bellows-like cylindrical shape centered on the central axis O. The upper end portion of the body portion 4c contacts the lower surface of the flange wall 25. The head portion 4a is movable in the up-and-down direction between the closing position and the opening position, that is, in the longitudinal direction of the spike 15 by elastic deformation of the body portion 4c in the up-and-down direction.

The engagement member 5 includes an engagement cylinder 36, two engagement claw portions 37, and two guided protrusions 38. The lower end portion of the engagement cylinder 36 forms a reduced diameter portion 36a having an inner circumferential surface that is reduced in diameter, the upper surface of the reduced diameter portion 36a contacts the lower surface of the shoulder portion 4b, and the inner circumferential surface of the reduced diameter portion 36a contacts the outer circumferential surface of the head portion 4a of the valve body 4. In addition, each of the two guided protrusions 38 protrudes radially outward from the outer circumferential surface of the engagement cylinder 36 at two positions facing each other in the first direction D1. Each of the guided protrusions 38 is disposed in a guide hole portion 39 provided on the inner circumferential surface of the upper cylindrical portion 30 and extending in the up-and-down direction, and is guided in the up-and-down direction by the guide hole portion 39. Each of the two engagement claw portions 37 protrudes radially outward from the outer circumferential surface of the engagement cylinder 36 at two positions facing each other in the second direction D2 and extends to the lower side, and has a distal end portion (lower end portion) bent radially inward. In addition, each of the two engagement claw portions 37 has a protruding portion 37a that protrudes radially outward. Each of the protruding portions 37a is disposed in the corresponding hole portion 35 at the closing position, and is pushed radially inward by the upper end edge portion of the hole portion 35 to elastically deform the engagement claw portion 37 radially inward when moving from the closing position to the opening position. Therefore, the engagement cylinder 36 is movable in the up-and-down direction integrally with the head portion 4a of the valve body 4 and relatively to the first connection member 2 and the second connection member 3.

As illustrated in Figs. 6 to 7, the second medical device 12 has a closing-type female connector portion 40 that can be mechanically connected to the second connection member 3 via the two locking claw portions 33a and fluidically connected to the second connection port 10 without intervention of the second connection member 3 by applying the second connection operation to the second medical device 12 by the relative parallel translation in the upward direction with respect to the medical connector 1. In addition, the second medical device 12 includes a male connector portion 41 having an internal passage that communicates with the internal passage of the female connector portion 40 on the opposite side of the male connector portion 40. Note that the second medical device 12 is not limited to such an I-shaped connector, and may be configured as, for example, another medical device having the female connector portion 40.

The female connector portion 40 has a cylindrical housing 42 and an on-off valve 43. The on-off valve 43 has a penetrating portion 43a penetrating the on-off valve 43 in the up-and-down direction at the central portion in top view. The penetrating portion 43a is constituted by, for example, a slit having a straight-line shape in top view. In addition, the on-off valve 43 has a clamped portion 43b at the outer circumferential edge portion in top view. The housing 42 includes an upper housing 42a and a lower housing 42b attached to the upper housing 42a, and clamps the clamped portion 43b in the up-and-down direction by the upper housing 42a and the lower housing 42b. The outer circumferential surface of the housing 42 has a circumferential groove 44 including two locked portions locked to the two locking claw portions 33a and two engaged portions engaged with the two engagement claw portions 37.

The second connection operation, by pushing up the top surface of the valve body 4 by the top surface of the female connector portion 40, allows the two engagement claw portions 37 to be engaged with the circumferential groove 44, further raised while maintaining the touching state between the top surfaces, allows the head portion 4a and the penetrating portion 43a of the on-off valve 43 to be penetrated by the distal end of the spike 15, the second connection port 10 to be communicated with the internal passage of the female connector portion 40, and allows the two locking claw portions 33a to be locked to the circumferential groove 44 by elastic deformation and restoration deformation. In this manner, by mechanically connecting the two locking claw portions 33a of the second connection member 3 to the female connector portion 40 of the second medical device 12 by the second connection operation, the second connection port 10 can be fluidically connected to the female connector portion 40 of the second medical device 12 without intervention of the second connection member 3.

According to the present embodiment, by applying the first connection operation, that is, the clockwise rotation operation in the bottom view, to the second connection member 3, the first connection member 2 can be co-rotated via the ratchet mechanism portion 8, and the first connection port 9 can be fluidically connected and the first connection member 2 can be mechanically connected to the first medical device 11.

In addition, according to the present embodiment, as illustrated in Figs. 6 to 7, even if an operation in a direction opposite to the first connection operation, that is, a counterclockwise rotation operation in a bottom view is unintentionally applied to the second medical device 12 or the second connection member 3 in a state where the medical connector 1 is connected to the first medical device 11 and the second medical device 12, the second connection member 3 can idle with respect to the first connection member 2 via the ratchet mechanism portion 8, so that it is possible to suppress unintentional detachment of the medical connector 1 from the first medical device 11.

The present disclosure is not limited to the above-described embodiment and may be modified in various manners without departing from the gist of the present disclosure.

Thus, the medical connector 1 according to the above-described embodiment can be changed in various manners as described below, for example.

The medical connector 1 according to the above-described embodiment can be changed in various manners as long as being the medical connector 1 including: the first connection member 2 having the first connection port 9 and the second connection port 10 that fluidically communicates with the first connection port 9; the second connection member 3; and the ratchet mechanism portion 8 that couples the first connection member 2 and the second connection member 3, in which the first connection operation by rotation is applied to the second connection member 3 to allow the first connection member 2 to co-rotate via the ratchet mechanism portion 8 to allow the first connection port 9 to be fluidically connected to the first medical device 11 and allow the first connection member 2 to be mechanically connected to the first medical device 11, the second connection member 3 is mechanically connected to the second medical device 12 by the second connection operation to allow the second connection port 10 to be fluidically connected to the second medical device 12 without intervention of the second connection member 3, and, an operation in a direction opposite to the first connection operation is applied to the second connection member 3 to allow the second connection member 3 to idle with respect to the first connection member 2 via the ratchet mechanism portion 8.

For example, the number of the engagement claw portions 37 is not limited to two, and may be one or three or more. A configuration without the engagement claw portions 37 may be adopted. The number of the locking claw portions 33a is not limited to two, and may be one or three or more. A configuration may be adopted in which the second connection member 3 is mechanically connected to the second medical device 12 by a configuration other than the locking claw portion 33a. The first connection member 2 is not limited to the configuration including the valve body 4. The first connection member 2 is not limited to the configuration including the spike 15. The first connection member 2 is not limited to the configuration including the female connector 13.

Note that, it is preferable that the medical connector 1 according to the above-described embodiment is the medical connector 1 in which the first connection member 2 includes the female connector 13 having the first connection port 9.

It is preferable that the medical connector 1 according to the above-described embodiment is the medical connector 1 in which the first connection member 2 includes the spike 15 having the second connection port 10.

It is preferable that the medical connector 1 according to the above-described embodiment is the medical connector 1 including the valve body 4 having the head portion 4a and the body portion 4c, in which the head portion 4a is movable in the longitudinal direction of the spike 15 between the closing position where the second connection port 10 is closed and the opening position where the second connection port 10 is opened by elastic deformation of the body portion 4c.

It is preferable that the medical connector 1 according to the above-described embodiment is the medical connector 1 having the engagement claw portion 37 that is pushed radially inward of the spike 15 and elastically deformed by moving integrally with the head portion 4a of the valve body 4 to the root side of the spike 15 and engages with the second medical device 12.

It is preferable that the medical connector 1 according to the above-described embodiment is the medical connector 1 in which the second connection member 3 includes the locking claw portion 33a capable of being mechanically connected to the second medical device 12 by elastic deformation.

### Reference Signs List

- 1: Medical connector
- 2: First connection member
- 3: Second connection member
- 4: Valve body
- 4a: Head portion
- 4b: Shoulder portion
- 4c: Body portion
- 5: Engagement member
- 6: Holder
- 7: Second connection member body
- 8: Ratchet mechanism portion
- 8a: Rotation coupling portion
- 8b: Rotation regulating portion
- 9: First connection port
- 10: Second connection port
- 11: First medical device
- 12: Second medical device
- 13: Female connector
- 13a: Male screw portion
- 14: Hollow shaft portion
- 15: Spike
- 16: Flange portion
- 17: Outer wall portion
- 17a: Upper wall portion
- 17b: Lower wall portion
- 18: Elastic deformation portion
- 18a: Elastic projecting piece
- 19: Inner cylindrical body
- 20: Outer cylindrical body
- 20a: Female screw portion
- 21: Syringe body
- 22: Male connector
- 23: Inner circumferential wall
- 24: Claw piece
- 25: Flange wall
- 26: Upper wall
- 26a: Stepped surface
- 27: Lower wall
- 28: Circumference groove
- 29: Ring-shaped uneven surface
- 29a: Protrusion
- 30: Upper cylindrical portion
- 31: Intermediate coupling portion
- 32: Lower cylindrical portion
- 33: Operation piece
- 33a: Locking claw portion
- 33b: Coupling portion
- 33c: Operation portion
- 34: Opening
- 35: Hole portion
- 36: Engagement cylinder
- 36a: Reduced diameter portion
- 37: Engagement claw portion
- 37a: Protruding portion
- 38: Guided protrusion
- 39: Guide hole portion
- 40: Female connector portion
- 41: Male connector portion
- 42: Housing
- 42a: Upper housing
- 42b: Lower housing
- 43: On-off valve
- 43a: Penetrating portion
- 43b: Clamped portion
- 44: Circumferential groove
- D1: First direction
- D2: Second direction
- O: Central axis

## Claims

1. A medical connector comprising:
a first connection member having a first connection port and a second connection port that fluidically communicates with the first connection port; a second connection member; and a ratchet mechanism portion that couples the first connection member and the second connection member, wherein
a first connection operation by rotation is applied to the second connection member to allow the first connection member to co-rotate via the ratchet mechanism portion to allow the first connection port to be fluidically connected and allow the first connection member to be mechanically connected to the first medical device,
the second connection member is mechanically connected to a second medical device by a second connection operation to allow the second connection port to be fluidically connected to the second medical device without intervention of the second connection member, and
an operation in a direction opposite to the first connection operation is applied to the second connection member to allow the second connection member to idle with respect to the first connection member via the ratchet mechanism.

2. The medical connector according to claim 1, wherein the first connection member includes a female connector having the first connection port.

3. The medical connector according to claim 1 or 2, wherein the first connection member includes a spike having the second connection port.

4. The medical connector according to claim 3, comprising a valve body having a head portion and a body portion, the head portion being movable by elastic deformation of the body portion in a longitudinal direction of the spike between a closing position where the second connection port is closed and an opening position where the second connection port is opened.

5. The medical connector according to claim 4, comprising an engagement claw portion that is pushed and elastically deformed radially inward of the spike by moving integrally with the head portion of the valve body on a root side of the spike and engages with the second medical device.

6. The medical connector according to any one of claims 1 to 5, wherein the second connection member has a locking claw portion capable of being mechanically connected to the second medical device by elastic deformation.
